# EUROPEAN PATENT APPLICATION

(11) **EP 4 566 587 A1**
(43) Date of publication of application: **11.06.2025**
(21) Application number: 23214863.5
(22) Date of filing: 07.12.2023
(51) Int. Cl.: A61K 8/98, A23L 33/19, A61P 17/00, A61P 17/12, A61Q 3/00, A61Q 5/00, A61Q 5/12, A61Q 19/00

(54) **CASHMERE GOAT MILK PROTEINS**

(71) Applicant: Onos Technologies Holding Limited, D02 F89 Dublin 2 (IE)
(72) Inventor: The designation of the inventor has not yet been filed
(74) Representative: Schwarz & Partner Patentanwälte GmbH

(57) **Abstract**

The present invention relates to the use of a composition comprising one or more proteins from cashmere goat milk as cosmetic, medicament and/or dietary supplement.

## Description

### TECHNICAL FIELD

The present invention relates to the field of milk proteins.

### BACKGROUND ART

Due to their unique properties, milk proteins have the potential to significantly improve the efficacy and quality of cosmetic and pharmaceutical formulations as well as of dietary supplements.

In recent years, the demand for natural and high-quality ingredients in the cosmetics industry, for instance, has increased significantly. Consumers are increasingly looking for products that are not only effective, but also gentle on the skin and manufactured in an environmentally friendly manner. Milk proteins offer a promising alternative to conventional chemical ingredients in this regard.

Milk proteins are rich in essential amino acids and comprise, when extracted from milk, vitamins and minerals, that are essential for the human and animal health. When applied to hair, for instance, it is known that milk proteins have moisturizing properties and can strengthen hair, give it shine and protect it from damage caused by environmental factors. In addition, studies have shown that milk proteins can have anti-inflammatory and soothing effects on the scalp.

However, previous approaches to using milk proteins in cosmetic products, for instance, have been limited and have not been able to exploit the full potential of these valuable ingredients. It is therefore an object of the present invention to provide novel formulation to maximize the efficacy of milk proteins.

### SUMMARY OF THE INVENTION

Hence, the present relates to use of a composition comprising one or more proteins from cashmere goat milk as cosmetic.

It turned surprisingly out that compositions comprising proteins from cashmere goat milk have significantly superior effects in cosmetic applications, for instance, compared to milk proteins of other sources. When applied to hair proteins from cashmere goat milk reduce significantly the formation of split ends and hair breakage. Split ends are points in the oldest part of the hair shaft, at the very bottom of each hair strand. Split ends typically occur when the ends of hair become dry and brittle. The use of cashmere goat milk proteins reduces significantly the formation of split ends. Furthermore, it could be shown that hair breakage can also be reduce by applying cashmere goat milk proteins on hair. Cashmere goat milk proteins have not only beneficial effects on hair but also on skin rendering the skin softer, reducing the formation of callus and dander. It could also be observed that cashmere goat milk proteins have also skin moisturizing effects.

Another aspect of the present invention relates to the use of a composition comprising one or more proteins from cashmere goat milk as a dietary supplement.

It surprisingly turned out that oral intake of proteins from cashmere goat milk have beneficial local effects in the gastrointestinal tract and beneficial systemic effects as well.

A further aspect of the present invention relates to a medicament comprising one or more proteins from cashmere goat milk.

It was also found that cashmere goat milk proteins can also be used as a medicament. Cashmere goat milk proteins topically applied, for instance, can be used in the treatment of skin diseases. Cashmere goat milk proteins have an anti-inflammatory effect, thus reducing inflammation processes either topically or when administered orally to a an individual or mammal.

Another aspect of the present invention relates to a composition comprising one or more proteins from cashmere goat milk to be used in the treatment of a skin disease or skin disorder.

### BRIEF DESCRIPTION OF THE FIGURES

Fig. 1 shows an electron microscopic image of a hair which has been washed with ethanol and dried.
Figs. 2A and 2B show electron microscopic images of a hair which has been washed with ethanol and dried and thereafter treated with cashmere goat cream at two different magnifications.
Fig. 3 shows an electron microscopic image of a hair which has been washed with ethanol and dried and thereafter treated with cashmere goat skim milk.
Fig. 4 shows an electron microscopic image of a hair which has been washed with ethanol and dried and thereafter treated with cream of milk of a grisons striped goat.
Fig. 5 shows an electron microscopic image of a hair which has been washed with ethanol and dried and thereafter treated with skimmed milk of a grisons striped goat.

### DESCRIPTION OF EMBODIMENTS

The composition of the present invention comprises one or more proteins from cashmere goat milk. "One or more proteins", as used herein, means that a fraction or substantially all proteins present in cashmere goat milk are part of the composition of the present invention. Methods for isolating or extracting proteins from milk are well known to the person skilled in the art.

The proteins may be obtained (isolated, extracted) from cashmere goat milk, cashmere goat milk cream or cashmere skim milk, which may comprise up to 4 wt% protein. According to a preferred embodiment of the present invention cashmere goat milk, cashmere goat milk cream or cashmere skim milk can be used directly in the composition of the present invention since they comprise the proteins of cashmere milk.

Methods for isolating and/or extracting proteins from milk are well known to the person skilled in the art. These methods involve separating the proteins from other components present in milk to obtain a concentrated protein solution or precipitate. Suitable methods may involve one or more of the following steps: milk collection; filtration to remove any solid particles or impurities; precipitation of the milk proteins (e.g. by adjusting the pH of the milk to a specific range using an acid, such as citric acid, or by adding an organic solvent like alcohol); separating the precipitated proteins from the liquid whey by centrifugation, decanting or filtration; protein purification using techniques like chromatography, ion exchange, or electrophoresis; and drying using methods like freeze-drying or spray-drying to obtain a stable powder form, which can be easily stored and transported.

According to a preferred embodiment of the present invention the cosmetic is a hair cosmetic, a nail cosmetic or a skin cosmetic.

Proteins from cashmere goat milk has excellent properties in the cosmetic treatment of hair, nails and skin. Hence, the cosmetic of the present invention can be a hair cosmetic, a nail cosmetic or a skin cosmetic. Depending on the type of cosmetic the composition comprising cashmere goat proteins may comprise further components.

The proteins from cashmere goat milk are the "active" ingredients in cosmetic compositions which can be used in the cosmetic treatment of hair, nails and skin, and are, thus, the key components responsible for the desired effects on hair, nails, or skin. For hair, further active ingredients include vitamins and botanical extracts that promote strength, growth, and shine. For nail treatments further ingredients like keratin, calcium and vitamins to strengthen and nourish nails can be used. Compositions used for skin treatments may include antioxidants, hyaluronic acid and/or retinol to address specific concerns like hydration, anti-aging or brightening.

Emollients and moisturizers can also be added as they help to hydrate and soften the hair, nails and/or skin. Examples include natural oils (such as jojoba oil, argan oil, or coconut oil), shea butter, glycerin, or silicone derivatives. Emollients create a protective barrier, preventing moisture loss and improving the overall texture and appearance.

Surfactants can be used in hair and/or skin treatments to cleanse and remove dirt, excess oil and impurities. They create lather and foam, enhancing the cleansing experience. Common surfactants include sodium lauryl sulfate, sodium laureth sulfate or cocamidopropyl betaine.

Conditioning agents added to said compositions help to improve the manageability, softness and/or smoothness of hair. Conditioning agents can include cationic surfactants like behentrimonium chloride or cetrimonium chloride, silicones like dimethicone or cyclomethicone, or natural ingredients like aloe vera or panthenol.

UV filters in products designed for sun protection are essential to shield the hair, nails or skin from harmful UV radiation. Common UV filters include titanium dioxide, zinc oxide, avobenzone and/or octinoxate.

To prevent microbial growth and extend the shelf life of cosmetic compositions, preservatives may be added. Examples of preservatives include parabens, phenoxyethanol or benzyl alcohol. It is important to choose preservatives that are effective yet safe for use.

Fragrances and colorants can be added to enhance the sensory experience of using the cosmetic compositions. Fragrances can be natural or synthetic, while colorants can be dyes or pigments approved for cosmetic use.

The specific combination and concentration of the aforementioned components may vary depending on the intended use and formulation type (e.g., cream, serum, lotion).

According to another preferred embodiment of the present invention the composition comprises native cashmere goat milk, preferably raw cashmere goat milk, a processed cashmere goat milk, a proteinaceous fraction of cashmere goat milk or a proteinaceous extract of cashmere goat milk.

It turned out that in principle the proteins of the cashmere goat milk can be obtained from raw or processed milk. It is just of importance that proteins of the cashmere goat milk are present in the composition of the present invention.

According to a further preferred embodiment of the present invention the processed cashmere goat milk is fermented cashmere goat milk and/or a precipitate of cashmere goat milk.

Fermented milk is produced by the action of specific bacteria or yeast cultures on milk, which leads to the conversion of lactose (milk sugar) into lactic acid. This process imparts a tangy flavor and changes the texture of the milk. Furthermore, if the pH in the fermented milk falls below a certain level, the proteins comprised therein precipitate. This allows obtaining fermented cashmere goat milk and/or a precipitate of cashmere goat milk to be used in the compositions of the present invention.

According to a preferred embodiment of the present invention the composition comprises 0.01 to 40 wt%, preferably 0.02 to 30 wt%, more preferably 0.1 to 20 wt%, more preferably 0.2 to 10 wt%, of the one or more proteins.

The composition of the present invention comprises at least 0.01 wt%, preferably at least 0.02 wt%, more preferably at least 0.1 wt%, more preferably at least 0.2 wt%, more preferably at least 0.5 wt%, more preferably at least 1 wt%, of cashmere goat milk proteins.

According to another preferred embodiment of the present invention the proteinaceous extract of cashmere goat milk is an organic solvent extract, preferably an alcoholic extract.

The proteins of the composition of the present invention may be obtained by extracting the proteins from cashmere goat milk or a faction of said milk (e.g. cream, skim milk) using an organic solvent. It is particularly preferred to use alcohol for the protein extraction. Methods for such extraction processes are well known to the person skilled in the art.

Since the compositions of the present invention can be used as or in a cosmetic, medicament or dietary supplement it is preferred to use organic solvents which can also be administered to humans or other mammals. This is advantageous if residual organic solvent cannot be entirely removed from the extract. Hence, it is preferred that the alcoholic extract is an ethanol extract.

According to another preferred embodiment of the present invention the proteinaceous extract of cashmere goat milk is obtainable by a method comprising the following steps:
a) adding an organic solvent to native cashmere goat milk, preferably raw cashmere goat milk,
b) isolating the proteins precipitated in step a), and
c) removing volatile substances from the isolated proteins of step b).

According to a preferred embodiment of the present invention the organic solvent used to obtain the proteinaceous extract is an alcohol, preferably ethanol.

According to another preferred embodiment of the present invention in step b) the precipitated proteins are isolated by filtration and/or centrifugation.

After precipitating the proteins the latter can be isolated by using various methods. Most preferred methods involve filtration and/or centrifugation. Filtration can be done using filter paper, for instance, Which may comprise cellulosic material. Centrifugation is done using centrifuges at at least 2000 rpm, preferably at least 5000 rpm.

According to a further preferred embodiment of the present invention in step c) volatile substances are removed from the isolated proteins of step b) by applying heat and/or by lyophilization.

According to another preferred embodiment of the present invention the isolated proteins in step c) are heated to 30°C to 70°C to remove volatile substances.

Lyophilization can be done using means and methods known in the art.

After step c) the isolated proteins are preferably granulated and/or pulverized.

Granulation and pulverization of the proteins of the cashmere goat milk is particularly advantageous because protein granules and protein powder can be safely stored without losing quality and by avoiding potential degradation of the proteins. Furthermore, granules and powder can used directly as cosmetic, medicament and/or dietary supplement or to manufacture the same by adding other compounds.

Obtaining a proteinaceous extract from milk using an organic solvent like ethanol (96%) is a promising method that is typically used in the food, pharmaceutical and cosmetic industries. The cashmere goat milk or a fraction of it may be subjected to preliminary treatments to remove impurities and ensure its safety for further processing. These treatments may include pasteurization, filtration, and clarification. Once the milk is prepared, the proteinaceous extract is obtained by utilizing an organic solvent, preferably ethanol (e.g. 96% v/v). The milk is mixed with the solvent in a controlled ratio, typically in the range of 1:1 to 1:5 (milk to ethanol volume ratio). The mixture is then subjected to gentle agitation or stirring to facilitate the extraction process. The extraction process is carried out preferably at a specific temperature, preferably around 4 to 10°C, to maintain the stability of the proteins and minimize degradation. The duration of the extraction process can vary depending on the desired yield and the specific proteins being targeted. Generally, an extraction time of 1 to 24 hours is sufficient to achieve a satisfactory proteinaceous extract.

After the extraction, the mixture is subjected to a separation step to separate the proteinaceous extract from the residual milk components and the solvent. This can be achieved through techniques such as centrifugation or filtration. The separated proteinaceous extract is then further processed to remove any remaining impurities and concentrate the proteins to the desired level.

According to another preferred embodiment of the present invention the cosmetic is in the form of a serum, a lotion, a cream, a gel, an ointment, a mousse or a cleaning agent for hair, nail and/or skin application.

Cosmetic formulations play a crucial role in the development of various personal care products, including lotions, creams, gels, ointments, mousses, and cleaning agents. These formulations are designed to enhance the appearance and health of the hair, nails, and skin.

Lotions are popular cosmetic formulations known for their lightweight and non-greasy texture. They are typically water-based and contain a combination of emollients, humectants, and active ingredients. Emollients, such as mineral oil or silicone derivatives, help to moisturize and soften the skin. Humectants, like glycerin or hyaluronic acid, attract and retain moisture, providing hydration. Active ingredients can vary depending on the desired effect, such as vitamins, antioxidants, or sunscreens.

Creams, on the other hand, are thicker and more occlusive than lotions. They are composed of a water phase and an oil phase, which are emulsified together using emulsifiers. The oil phase consists of oils or fats, such as mineral oil or shea butter, providing nourishment and creating a protective barrier on the skin. The water phase contains water and humectants, ensuring hydration. Creams often include additional active ingredients, such as retinol or peptides, targeting specific skin concerns.

Gels are transparent or translucent formulations with a jelly-like consistency. They are water-based and contain gelling agents, such as carbomer or xanthan gum, to create the gel texture. Gels are commonly used for their cooling and soothing properties. They can contain active ingredients like aloe vera or tea tree oil, known for their calming effects on the skin.

Ointments are semi-solid formulations with a greasy texture. They are typically anhydrous, meaning they do not contain water. Ointments are composed of a base, such as petrolatum or lanolin, which provides occlusion and moisture retention. They are particularly suitable for dry or damaged skin. Ointments can include active ingredients like corticosteroids or antibiotics for medicinal purposes.

Mousses are lightweight and foamy formulations that are often used for hair styling or body application. They are typically aerosol-based and contain propellants to create the foam texture. Mousses can contain polymers, such as polyquaterniums, which provide hold and volume to the hair. They may also include conditioning agents like panthenol or silk proteins for added benefits.

Cleaning agents for hair, nails, and skin are formulated to remove dirt, oil, and impurities. These formulations can vary depending on the specific application. For hair, cleansing agents like surfactants, such as sodium lauryl sulfate or cocamidopropyl betaine, are commonly used. Nail cleaning agents may contain solvents like acetone or ethyl acetate to remove nail polish or debris. Skin cleaning agents can include gentle surfactants like sodium cocoyl isethionate or natural exfoliants like jojoba beads.

Another aspect of the present invention relates to the use of a composition comprising one or more cashmere goat milk proteins as defined and described herein as a dietary supplement.

Dietary supplements of the present invention comprise one or more cashmere goat milk proteins. Dietary supplements of the present invention may have various dosage forms.

Milk protein powders are a popular dosage form for dietary supplements. They can be easily mixed with water or other beverages, making them convenient for consumption. Powders can be formulated to contain specific ratios of milk proteins, catering to different nutritional needs and goals. Powders may comprise 1 wt% to 100 wt%, preferably 2 wt% to 95 wt%, more preferably 5 wt% to 80 wt%, of the cashmere milk proteins of the present invention.

Milk protein-based dietary supplements can also be formulated in the form of capsules or tablets. These dosage forms offer convenience and precise dosing, allowing users to easily incorporate them into their daily routine.

Dietary supplements in the form of capsules and tablets have gained popularity as convenient and effective ways to support overall health and well-being. These two formulations offer distinct advantages and are widely used due to their ease of consumption and precise dosage delivery. Let's delve into the details of how capsules and tablets are formulated to provide the desired benefits.

Capsules, typically made of gelatin or vegetarian alternatives, consist of two halves that enclose the supplement's contents. The formulation process begins with carefully selecting the active ingredients, which can include vitamins, minerals, herbs, or other bioactive compounds. These ingredients are then blended and mixed to ensure a homogeneous distribution.

To create the capsule, the powdered or liquid supplement mixture is filled into the bottom half of the capsule shell. The top half is then placed on top, and the two halves are sealed together. This encapsulation process protects the ingredients from degradation, moisture, and external factors, ensuring their stability and potency until consumption.

Capsules offer several advantages. They are generally easier to swallow due to their smooth and slippery texture. Additionally, the encapsulation process allows for the inclusion of oil-based or liquid ingredients that may not be feasible in tablet formulations. Moreover, capsules can be designed to release their contents at specific locations in the digestive system, ensuring optimal absorption and bioavailability.

On the other hand, tablets are solid, compressed forms of dietary supplements. The formulation of tablets involves carefully blending the active ingredients with excipients, which are substances that aid in the tablet's formation and stability. Excipients can include binders, disintegrants, lubricants, and fillers.

The blend is then compressed under high pressure into a tablet shape using specialized machinery. Tablets can be coated to improve appearance, taste, and ease of swallowing. Coatings can also provide additional protection against moisture and enhance stability.

Tablets offer advantages such as convenience, as they are easy to handle and transport. They also have a longer shelf life compared to capsules. Moreover, tablets can be formulated to release their contents at different rates, allowing for sustained or delayed release formulations.

Milk protein bars have gained popularity as a convenient on-the-go option for individuals seeking a quick and nutritious snack. These bars can be formulated to contain milk proteins along with other essential components, providing a balanced nutritional profile.

In addition to protein, dietary supplement bars are often fortified with vitamins and minerals. These micronutrients play a vital role in maintaining overall health and well-being. Commonly included vitamins and minerals in these bars include vitamin C, vitamin D, vitamin B complex, calcium, iron, and zinc. These nutrients help to fill potential gaps in the diet and support various bodily functions.

To enhance the taste and texture of the bars, they may also contain carbohydrates and healthy fats. Carbohydrates provide a quick source of energy, making them beneficial for individuals engaging in physical activities or needing an energy boost. Healthy fats, such as those derived from nuts, seeds, or oils, contribute to the overall nutritional profile of the bars and provide essential fatty acids.

Furthermore, dietary supplement bars may incorporate additional functional ingredients. These can include fiber, probiotics, antioxidants, or herbal extracts. Fiber aids in digestion and promotes a feeling of fullness, while probiotics support gut health. Antioxidants help to combat oxidative stress in the body, and herbal extracts may offer specific health benefits depending on the ingredient used.

Ready-to-Drink (RTD) beverages are another dosage form for milk protein-based dietary supplements. These beverages offer convenience and are often fortified with additional vitamins, minerals, and other essential components to provide a complete nutritional package.

Cashmere milk protein-based dietary supplements may include one or more of the following components. Milk protein-based dietary supplements can be enriched with essential vitamins and minerals to support overall health and well-being. These may include but are not limited to vitamin D, calcium, iron, and zinc. Incorporating omega-3 fatty acids, such as EPA and DHA, into milk protein-based dietary supplements can provide additional cardiovascular and cognitive benefits. Adding dietary fiber to these supplements can promote digestive health and aid in weight management. Probiotics are beneficial bacteria that can support gut health and enhance the immune system. Including probiotics in milk protein-based dietary supplements can provide added health benefits.

A further aspect of the present invention relates to a medicament comprising one or more proteins from cashmere goat milk, preferably as defined above.

The composition of the present invention can be used as a medicament for various topical and systemic diseases and disorders since the proteins of cashmere goat milk show surprisingly anti-inflammatory properties, for instance. In particular the beneficial effects of cashmere goat milk proteins on the skin allow the use of the composition of the present invention in the treatment of a skin disease or skin disorder.

Skin diseases pose significant challenges to patients and healthcare providers worldwide. The development of effective treatment options is crucial to alleviate symptoms and improve patients' quality of life. This patent application focuses on the formulation of hydrogels, ointments, emulsions, and creams, which have shown promising results in treating various skin diseases.

Hydrogels are three-dimensional networks of hydrophilic polymers that can absorb and retain large amounts of water. They provide a moist environment, promoting wound healing and drug delivery. Common components in hydrogels include polymers like polyvinyl alcohol, polyethylene glycol, and hyaluronic acid. These polymers enhance the gel's stability, viscosity, and milk protein release properties.

Ointments are semi-solid preparations primarily composed of hydrophobic substances. They provide occlusion, hydration, and protection to the skin. Key components in ointments include petrolatum, mineral oil, lanolin, and various waxes. These ingredients help retain moisture, enhance drug penetration, and provide a protective barrier against external irritants.

Emulsions are biphasic systems consisting of two immiscible liquids, typically oil and water, stabilized by an emulsifying agent. They offer versatility in delivering both hydrophilic and lipophilic drugs. Common components in emulsions include emulsifiers like lecithin, sorbitan esters, and polyethylene glycol derivatives. These emulsifiers stabilize the system, ensuring uniform milk protein dispersion and controlled release.

Creams are semi-solid emulsions with a balanced ratio of oil and water. They provide a smooth and elegant texture, making them suitable for topical application. Components in creams include emulsifiers, thickeners, and humectants. Emulsifiers like cetyl alcohol and stearic acid stabilize the oilwater interface, while thickeners like carbomer enhance viscosity. Humectants like glycerin and propylene glycol retain moisture, preventing skin dryness.

The combination of hydrogels, ointments, emulsions, and creams offers a wide range of treatment options for various skin diseases. These formulations can be tailored to address specific conditions such as eczema, psoriasis, acne, and dermatitis. Additionally, active pharmaceutical ingredients (APIs) like corticosteroids, antibiotics, antifungals, and immunomodulators can be incorporated into these formulations to provide targeted therapy.

Exemplary skin diseases or skin disorders are selected from the group consisting of pityriasis capitis, tinea capitis and psoriasis.

Pityriasis capitis, also known as dandruff, is a common scalp condition characterized by flaking and itching of the scalp. It is often caused by an overgrowth of a yeast-like fungus called Malassezia. Current treatment for pityriasis capitis typically involves the use of over-the-counter medicated shampoos containing active ingredients such as zinc pyrithione, ketoconazole, or selenium sulfide. These shampoos are usually used 2-3 times per week or as directed by a healthcare professional. However, these conventional methods do not provide sufficient relief. In contrast thereto cashmere milk proteins are able to treat pityriasis capitis efficiently and long-lasting.

Tinea capitis, commonly known as scalp ringworm, is a fungal infection of the scalp. It is more common in children but can affect people of all ages. Treatment for tinea capitis usually involves the use of antifungal medications, both topical and oral. Topical antifungal creams or ointments containing ingredients like clotrimazole or terbinafine may be prescribed for mild cases. Oral antifungal medications, such as griseofulvin or terbinafine, may be necessary for more severe or widespread infections. The dosing and dosing intervals for these medications will vary depending on the specific medication and the severity of the infection. Cashmere milk proteins allow to avoid antibiotics since these proteins are surprisingly helpful in the treatment of tinea capitis.

Psoriasis is a chronic autoimmune skin condition that can affect various parts of the body, including the scalp. Scalp psoriasis is characterized by red, scaly patches on the scalp, which can be itchy and uncomfortable. Treatment for scalp psoriasis often involves the use of topical corticosteroids, which help reduce inflammation and itching. These medications are available in different strengths and formulations, and the dosing and dosing intervals will depend on the severity of the condition and the specific medication prescribed. Other treatment options for scalp psoriasis may include coal tar preparations, salicylic acid, or calcipotriene. Cashmere milk proteins allow to avoid therapeutics like corticosteroids having unwanted side effects since these proteins are surprisingly helpful in the treatment of psoriasis. Cashmere milk proteins do not show any side effects when administered to an individual.

Preferred pharmaceutical compositions may comprise 1 to 50 wt%, preferably 2 to 40 wt%, more preferably 5 to 30 wt%, cashmere milk proteins and may be applied one to five times a day, preferably one to five times a week.

The present invention is further illustrated in the following examples, however, without being restricted thereto.

### EXAMPLES

In the examples the use of proteins obtained from the milk of cashmere goats for applications in medicine, as food supplements and in cosmetics is shown.

All the experiments provided herein clearly show that proteins from the milk of cashmere goats are particularly suitable for repairing pre-existing hair damage. They also show effectiveness in skin healing processes on the scalp and in wound healing.

Cashmere goats are known for their undercoat, which is used to make cashmere yarns. This undercoat is naturally produced seasonally by the animal and is produced by a combination of enzymes and proteins. The undercoat is characterized by its particular softness, due to the very smooth surface of the individual hairs.

Mother animals during the season for harvesting the undercoat do not show this special softness, and their undercoat is rougher. This can also be seen under the electron microscope. Fawns, on the other hand, have very soft hair overall, and smooth surfaces can be seen under the electron microscope.

It can be concluded from this that the necessary enzymes and proteins are passed on to the fawn via the mother's milk. It is therefore considered proven that the described proteins are absorbed via the gastrointestinal tract and play an essential role in the formation of the soft coat.

### Example 1: Cashmere goat milk proteins for hair repair under the electron microscope

In this example, 1 litre of raw milk was milked directly from cashmere goats. The cream was separated from the skim milk.

In addition, 1 litre of raw milk each from a grisons striped goat ("Bündner Strahlenziege") and a peacock goat were milked as comparison samples. Here, too, the cream was separated from the skim milk.

For the treatment, an auburn hair bundle from a female was cut at the lower end of the hair. The hair showed significant hair damage in the form of split ends and felt rough.

The hair bundle was soaked in 98% ethanol for 24 hours and shaken before treatment to wash away grease, surfactants, impurities and proteins. This process was repeated several times until the ethanol was clear and residue-free.

The hair bundle was then dried on a sterile screen. The hair was then placed in 5 sample vials:
Sample 1: hair without treatment, control sample
Sample 2: hair treated with cream of cashmere goat milk
Sample 3: hair treated with skim milk of cashmere goat milk
Sample 4: hair treated with cream of grisons striped goat
Sample 5: hair treated with skimmed milk of grisons striped goat

The samples, except for the control sample, were each soaked in the corresponding milk/cream samples for 20 minutes. They were then shaken over a shaker for 10 minutes.

The hairs were then removed and dried over a sterile sieve using a hair dryer. The hair ends were visually inspected for split ends. The initial visual evaluation yielded the following findings:
Sample 1 (control sample): hair split clearly visible
Sample 2: hair split not detectable
Sample 3: hair split not detectable
Sample 4: hair split clearly visible
Sample 5: hair split clearly visible

All specimens were subsequently trimmed to 0.5 cm length and examined under an electron microscope.

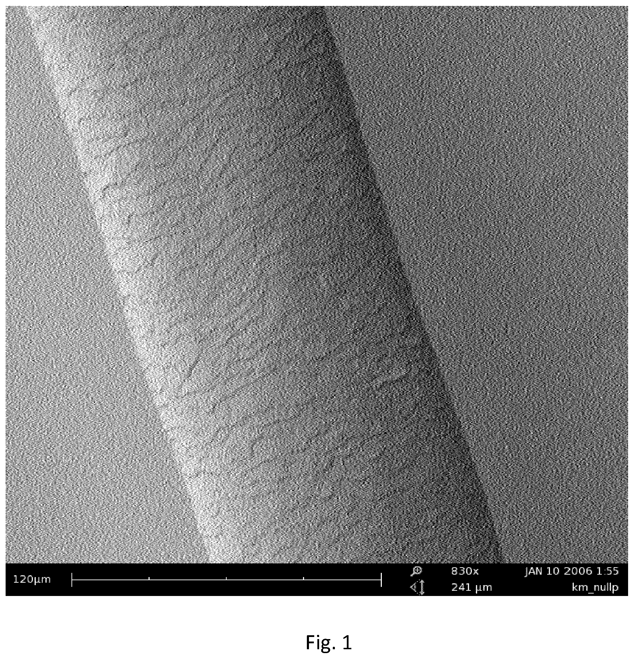

Fig. 1 shows an electron microscopic image of a hair of the control sample. Cracks can be seen on the surface, which are known to be common in all human hair. These structures are well known.

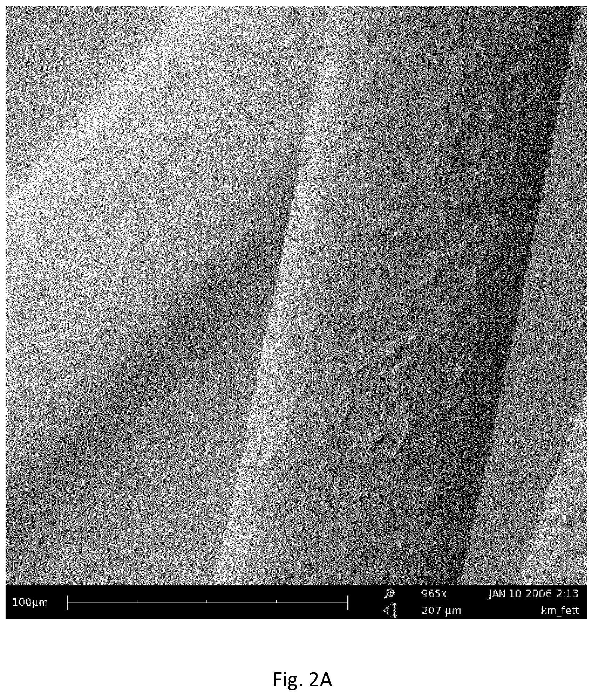

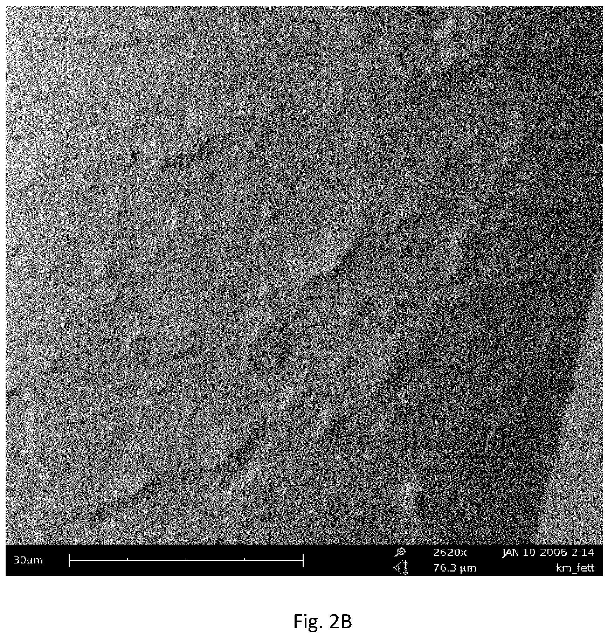

Fig. 2 shows an electron microscopic image of hair treated with cashmere goat cream (sample 2). The images show a significant improvement in the surface structure. The surfaces were smoothed by the proteins present in the cashmere goat cream (see Fig. 2A). Even at a higher magnification of 76.3 µm the surface shows no cracks in comparison to the control sample (see Fig. 2B). This explains the soft and silky feel of the hair when treated with cashmere goat cream.

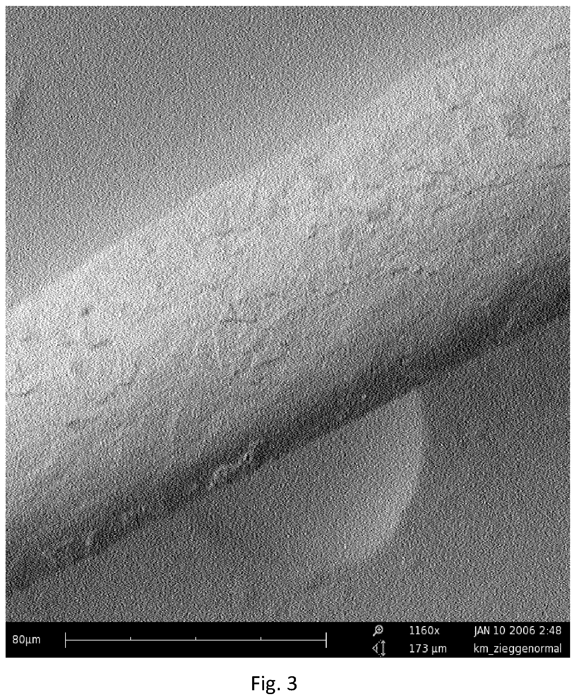

Fig. 3 shows hair treated with cashmere goat skim milk. The images show the same results as for sample 2 (treated with cashmere goat cream; Fig. 2).

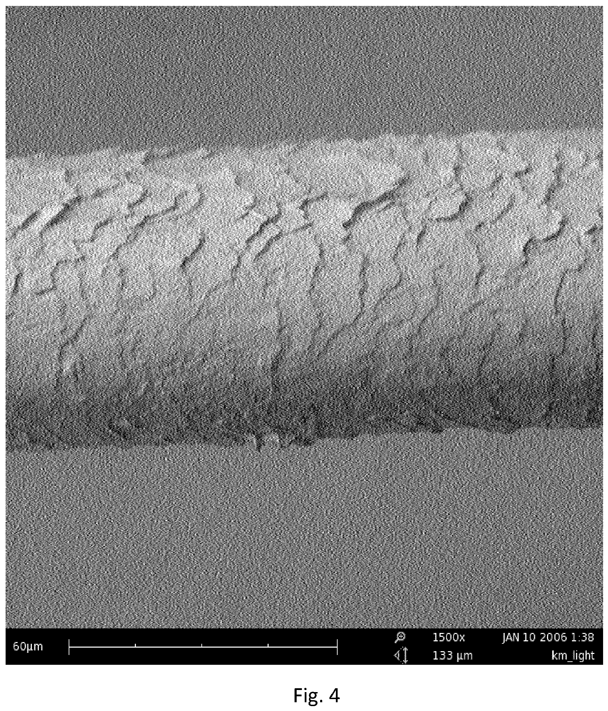

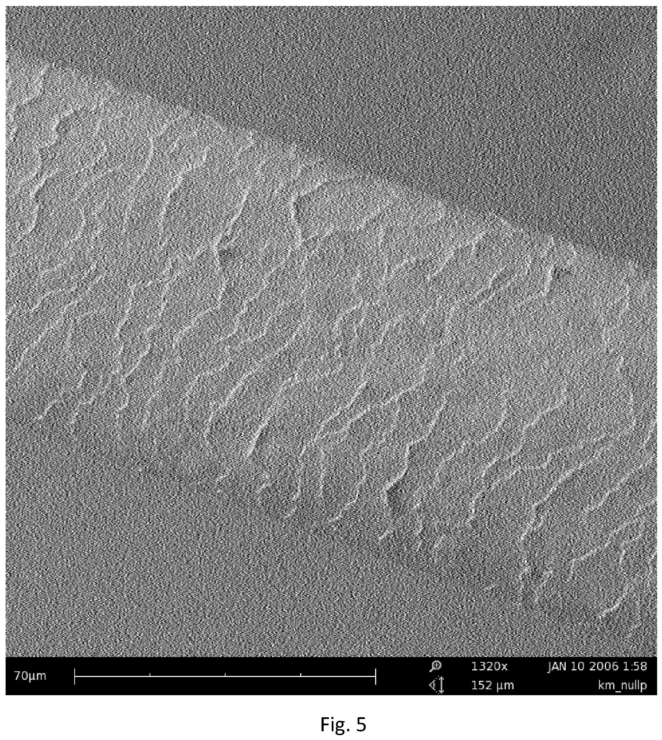

Figs. 4 and 5 show hair samples treated with cream of milk and skimmed milk of grisons striped goat, respectively. It can be seen that the surface of both hair samples is similar to the hair sample of the control (sample 1). This clearly shows that goat milk components from goats other than cashmere goat have nearly no effect on the hair structure. The same results have been obtained by treating hair samples with cream and skimmed milk of peacock goats.

In a comparative study the protein fraction has been removed from the cashmere goat cream and the cashmere goat skim milk by precipitation. Both of these deproteinized compositions have been applied on hair samples as described above. It turned surprisingly out that the deproteinized compositions had the effect on hair as the control sample as described above.

### Example 2: Effects of cashmere goat milk proteins on pityriasis capitis

A patient suffering from pityriasis capitis and severe itching was given petrolatum as a control. Petrolatum was applied twice weekly to the scalp and allowed to act for 10 minutes. After 4 weeks of treatment no improvement occurred, dandruff remained unchanged, and itching of the scalp persisted.

The same patient was then given a pre-made mixture comprising 90% petrolatum and 10% proteins isolated from cashmere goat's milk. These proteins were obtained from the skim milk by adding 50% ethanol (98%). The precipitated proteins were filtered, dried, grounded, and dispersed into the petrolatum with a dissolver.

The patient applied the mixture to the scalp twice a week. After 4 weeks of treatment dandruff forming had stopped and itching had disappeared after the first application of the mixture.

### Example 3: Treatment of corns and calluses

Corns and calluses are caused by pressure or friction on skin. A corn is thickened skin on the top or side of a toe. Most of the time it is caused by bad-fitting shoes. A callus is thickened skin on hands or the soles of feet. The thickening of the skin is a protective reaction. Corns and calluses may be painful and may bleed.

The petrolatum mixture comprising 10% proteins isolated from cashmere goat's milk of example 2 was given to a patient who had severe callus formation on his hands. After using it three times a day for two weeks, a significant reduction in callus was noted. Previous treatments with petrolatum alone had no effect on callus formation on his hands.

### Example 4: Food supplements

One patient was given one tablespoon of protein powder (approx. 2 g) dissolved in 5 to 10 ml water daily for a period of 3 months. The protein powder was obtained from the skim milk of cashmere goats by mixing it with 50% ethanol (98%). The precipitated proteins were filtered, dried, grounded and given to the patient (200g).

The patient reported an improvement in his skin appearance as well as his hair and a general better feeling of well-being, especially in the gastrointestinal tract.

### Example 5: Cosmetic product

Cream from the cashmere milk was whipped into butter and then gently cooked at 70°C. This formed clarified butter, which was filtered. With the addition of NaOH, the clarified butter was converted into a soap. For this purpose, 1000g of clarified butter was melted at 30°C. Separately, 162g of NaOH was dissolved in 50g of water. The NaOH solution was added to the liquid clarified butter and mixed until a soap was formed. The still liquid soap was poured into silicone containers and then stored for 4 weeks to fully react.

The soaps were distributed to 10 different people for testing. The soap was used for both body and hair. All test subjects reported a significant improvement in skin texture and particularly soft, supple and silky shiny hair. The hair was particularly easy to comb and split ends were no longer present.

## Claims

1. Use of a composition comprising one or more proteins from cashmere goat milk as cosmetic.

2. The use of claim 1, wherein the cosmetic is a hair cosmetic, a nail cosmetic or a skin cosmetic.

3. The use according to claim 1 or 2, wherein the composition comprises native cashmere goat milk, preferably raw cashmere goat milk, a processed cashmere goat milk, a proteinaceous fraction of cashmere goat milk or a proteinaceous extract of cashmere goat milk.

4. The use according to claim 3, wherein the processed cashmere goat milk is fermented cashmere goat milk or a precipitate of cashmere goat milk.

5. The use according to any one of claims 1 to 4, wherein the composition comprises 0.01 to 40 wt% of the one or more proteins.

6. The use according to any one of claims 3 to 5, wherein the proteinaceous extract of cashmere goat milk is an organic solvent extract, preferably an alcoholic extract, more preferably an ethanol extract.

7. The use according to any one of claims 3 to 6, wherein the proteinaceous extract of cashmere goat milk is obtainable by a method comprising the following steps:
a) adding an organic solvent to native cashmere goat milk, preferably raw cashmere goat milk,
b) isolating the proteins precipitated in step a), and
c) removing volatile substances from the isolated proteins of step b).

8. The use according to claim 7, wherein in step c) volatile substances are removed from the isolated proteins of step b) by applying heat and/or by lyophilization.

9. The use according to claim 8, wherein the isolated proteins in step c) are heated to 30°C to 70°C to remove volatile substances.

10. The use according to any one of claims 7 to 9, wherein after step c) the isolated proteins are granulated and/or pulverized.

11. The use according to any one of claims 1 to 13, wherein the cosmetic is in the form of a serum, a lotion, a cream, a gel, an ointment, a mousse or a cleaning agent for hair, nail and/or skin application.

12. Use of a composition as defined in any one of claims 1 to 10 as a dietary supplement.

13. Medicament comprising one or more proteins from cashmere goat milk, preferably as defined in any one of claims 3 to 10.

14. Composition as defined in any one of claims 3 to 11 to be used in the treatment of a skin disease or skin disorder.

15. Compositions according to claim 14, wherein the skin disease or skin disorder is selected from the group consisting of pityriasis capitis, tinea capitis, and psoriasis.
